# EUROPEAN PATENT SPECIFICATION

(11) **EP 4 166 100 B1**
(45) Date of publication and mention of the grant of the patent: **03.12.2025**
(21) Application number: 22200838.5
(22) Date of filing: 11.10.2022
(51) Int. Cl.: A61B 17/322, A61B 90/00

(54) **DERMATOME BLADE UNIT, KIT OF SUCH UNITS, DERMATOME COMPRISING SUCH A UNIT, AND KIT OF PARTS OF SUCH DERMATOMES**
DERMATOMKLINGENEINHEIT, KIT AUS SOLCHEN EINHEITEN, DERMATOM MIT SOLCH EINER EINHEIT UND KIT AUS TEILEN SOLCH EINER DERMATOM
UNITÉ DE LAME DE DERMATOME, KIT DE TELLES UNITÉS, DERMATOME COMPRENANT UNE TELLE UNITÉ, ET KIT DE PARTIES DE TELS DERMATOMES

(30) Priority: 14.10.2021 NL 2029404
(43) Date of publication of application: 19.04.2023
(73) Proprietor: Humeca B.V., 7623 CS Borne (NL)
(72) Inventor: Haagsma-van der Kouwe, Daniëlle Elisabeth, 7623 ZC Borne (NL)
(74) Representative: Arnold & Siedsma

(56) References cited:
- US-A1- 2009 157 095

## Description

The present invention relates to dermatomes. In particular, the invention relates to dermatomes having single use parts that are releasably connectable to a reusable part.

Dermatomes as such are known. Dermatomes are used to harvest skin grafts from a human or animal body. The skin graft can then be used to treat wounds, for instance burn wounds, at which no skin or damaged skin may be present. Normally, skin is harvested from the person to be treated. Accordingly, the such a grafting process is called autografting.

In general dermatomes consist of a handle and a head, wherein the head comprises a blade which in most cases is driven by some kind of drive means. Blades are single use. Accordingly, the dermatome head comprises a receiving structure for receiving and holding the blade at a certain position. The head further comprises a guide surface, which in use of the dermatome slides across the skin of the donor. A position of the blade with respect to the guide surface, which is called the blade height or cutting height, defines the position of the blade with respect to the surface of the skin when the dermatome is used. Accordingly, a thickness of the skin grafts is determined by said blade height. In order to allow a user, such as a doctor, to select a desired thickness for the skin grafts, dermatomes may be provided with a blade height adjustment mechanism, which moves the receiving structure in order to move the blade with respect to the guide surface.

An example of a dermatome is known from US 2009/0157095 A1. It describes a dermatome with a control bar, but also with interchangeable blade assemblies in paragraphs [0042] - [0044].

Although the existing dermatomes are satisfactory to some extent, a need for improvement remains. In particular, dermatomes that allow blade height adjustment are notoriously hard to clean due to their many, possibly small, movable parts. Accordingly, such dermatomes pose a serious health risk through a possible lack of hygiene, which could lead to e.g. cross contamination or which could facilitate infections of the skin graft and the wound to be treated, and/or of the donor site, the donor site being a part of the body from which the skin graft is taken.

The invention aims to provide an improved dermatome solution, in particular one which does not pose the same health risks.

The aim is achieved by a dermatome blade unit as defined in the claims, a kit of replaceable dermatome blade units as defined in the claims, and by a dermatome and a kit of parts as defined in the claims.

Advantages of the dermatome blade unit are described below.

If the attachment height of first and second dermatome blade unit provided in a kit differs, the blade height when the dermatome blade units are connected to a dermatome differs between the two dermatome blade units. Accordingly, the blade units can be used to produce skin grafts of different thicknesses. At the same time, the added complexity of a blade height adjustment mechanism in the head of the dermatome is avoided. Accordingly, the dermatome may be easier to clean, thereby reducing health risks.

The invention stems in part from the realization that a health advantage may be achieved by moving complexity from the dermatome head to the blade unit, since the blade unit is single use and the dermatome head needs to be cleaned for reuse. Accordingly, less complex parts need to be cleaned.

Moreover, since the blade height and thus the graft thickness is defined by the blade unit, it is no longer possible inadvertently select a wrong setting on the dermatome, which may occur e.g. during use when a set height adjustment mechanism fails and changes the blade height setting unexpectedly. According to the invention, failure of the height adjustment can be avoided, which may reduce a chance of medical errors. Additionally or alternatively, the blade height used can be easily reviewed before and even after using the dermatome by checking the blade unit used. Accordingly, the grafting operation becomes less error prone and more traceable.

It is noted that the blade and the blade support or parts thereof may be integrally made, i.e. they may be formed of the same, single part. As an alternative, they may be made of different parts, which are optionally movable with respect to each other. In particular, the blade may comprise a metal, whereas the blade support may comprise at least one plastic part.

A relatively elegant design of the dermatome blade unit may be achieved if the dermatome blade unit is connectable to a head of a dermatome, which may be reusable. As explained above, the dermatome head may accordingly be easier to clean.

However, the invention also finds application in case the dermatome blade unit is comprised by a dermatome head, which may thus be replaceable and/or single use in its entirety. In that case, the enhanced traceability and reduced chances of medical errors remain important advantages achieved through the invention.

It is noted that it is not strictly necessary to provide blade units having a different attachment height. Some advantages of the invention as described herein may be achieved with dermatome blade units having the same or similar attachment heights.

In an embodiment of the dermatome blade unit, the blade support comprises a cover and a clamping element, the blade being interposed between the cover and the clamping element.

Interposing the blade between a cover and clamping element may allow securely holding the blade, especially when allowing movement of the blade with respect to the cover and/or clamping element. The cover may at the same time be used to cover a bottom of the dermatome head. Moreover, the blade units according to this embodiment may be relatively easy to produce.

It is noted prior art dermatomes also include a cover, in order to at least partly cover the dermatome blade, and other (moving) parts of the dermatome. Such covers are fixed to the dermatome head using e.g. bolts. By including the cover in the dermatome blade unit, additional complexity is removed from the dermatome, which may therefore be cleaned more easily.

The connecting means may be fixed to or may be part of one or both of the clamping element and the cover. In particular, the connecting means are an integral part of the cover. In that case, the clamping element may be used to lock the blade in place on the cover. The clamping element may be connected to the cover via a clamping connection, for instance as described below. However, it is also possible to connect the clamping element to the cover in some other way, as long as the blade is kept in place. As such, the clamping element need not necessarily clamp. In fact, the clamping element may therefore also be replaced by a locking element or some other component.

There is also the possibility that the blade support only comprises one of a cover and a clamping element. For instance, the blade support may comprise a cover and no clamping element. In that case the blade would be held by the cover, optionally in combination with another component of the dermatome, such as a component of the dermatome head. Another option is that the blade support comprises a clamping element, or some other kind of element fixing the dermatome blade, wherein said element optionally cooperates with a part of the dermatome, such as a blade cover attached to the dermatome head, in order to (movably) keep the dermatome blade in its place.

In yet another embodiment of the dermatome blade unit, one of the cover and the clamping element comprises male connecting units and the other of the cover and the clamping element comprises corresponding female connecting units, the male and female connecting units being configured to engage each other in order to lock the clamping element in place with respect to the cover.

By locking the clamping unit in place with respect to the cover, the blade may be held securely between the two. Using male and corresponding female connecting units may be a practical way to provide the desired locking action, although alternatives may also be used.

The male connecting units may comprise one or more pillars, extending from in particular the cover. The female connecting units may comprise apertures or recesses, in particular in the clamping element. The apertures or recesses and the pillars may be shaped so as to allow introduction of the pillars into the apertures or recesses under plastic deformation of the pillars and/or apertures or recesses, thereby clamping the apertures or recesses onto the pillars. As such, a clamping connection between the cover and the clamping element may be made.

The cover may be provided with one or more stops, for instance at the pillars, for engaging the clamping element. The one or more stops may be placed so that when the clamping element engages the stop(s), the cover and clamping element together define a suitable receiving space for the blade. The stop(s) may be arranged so that the blade has a certain play in a direction from the clamping element to the cover and vice versa, so as to allow movement of the blade in a direction transversal to the mentioned direction. Of course the stop(s) may alternatively or additionally be arranged on the clamping element.

The dermatome blade of a dermatome blade unit is moveable with respect to the respective blade support in a direction substantially parallel to a direction in which a cutting edge of the dermatome blade extends.

This movement of the blade, which is transversal with respect to the grafting direction causes the blade to cut more easily into or through the skin. Accordingly, less force needs to be produced when grafting and/or cleaner cuts may be made. Moreover, allowing motion of the blade with respect to the blade support allows to keep the blade support stationary with respect to the dermatome and/or dermatome head. As such, the dermatome or dermatome head may be designed more elegantly, as no or little moving parts are needed.

In yet another embodiment of the dermatome blade unit, the dermatome blade comprises an engagement surface, configured for engagement by drive means of a dermatome for moving the dermatome blade with respect to the blade support.

Motion of the blade, for instance that described in the preceding paragraphs, may be realized by engaging the blade by the drive means to this engagement surface.

When the blade unit is attached to the dermatome head, and a handle is attached to the head, the a drive means arranged in the handle may engage directly on the engagement surface of the blade unit. Accordingly, the head can be kept free of driving and/or driven components, so that the head can be designed relatively elegant and/or with few components. Accordingly, the head may be more easy to clean.

In accordance therewith, the handle may comprise a drive means. The drive means may comprise an output. The head may comprise a through feed for the output of the drive means to run towards the blade unit. The blade unit may comprise the aforementioned engagement surface. The output of the drive means may therefore engage on the blade unit through the head, without actually requiring components of the head to be part of the drive train. It is of course possible to, as an alternative, provide a coupling as part of the head, which receives input from the output of the drive means and engages on the engagement surface of the blade unit. This however complicates design of the dermatome head.

The capability to drive the blade unit with the drive means in the handle directly, is especially advantageous when the blade unit and/or the head can be decoupled / detached from the handle and from each other. In such a case, the head and handle may be combined as desired, for instance by selecting a desired width of the head. Yet the head and the handle may be cleaned relatively easily when the head is detached from the handle.

In another embodiment of the dermatome blade unit, the engagement surface is formed by an aperture and/or recess in the dermatome blade.

Via the aperture and/or recess the blade can be pushed in a direction parallel to the plane of the blade, while motion of the blade in a direction normal to the plane may be decoupled from motion of the drive means in that direction. Accordingly, unwanted vibrations and/or motion in the normal direction may be at least partially avoided.

In yet another embodiment of the dermatome blade unit, the blade support comprises a guide surface for guiding the dermatome blade unit across skin during use of the respective dermatome blade unit in a dermatome.

The guide surface may allow smooth movement of the dermatome over the skin, for instance at a well defined height, and therefore give better control when using the dermatome.

The guide surface of the blade unit may be flush with an additional guide surface of the dermatome head.

As the guide surface defines the position of the dermatome with respect to the skin, the position of the connecting means with respect to the guide surface of the blade unit and/or the dermatome head may vary from dermatome blade unit to dermatome blade unit, in correspondence with the attachment height. As such, the varying attachment height may also be described as a varying distance from blade to guide surface, as seen in a thickness direction of a skin graft to be cut. The thickness direction can be inferred from the orientation and position of the guide surface and the blade, and may for instance be substantially normal to a plane of the blade.

In yet another embodiment of the dermatome blade unit, the dermatome blade unit comprises a removable blade protector, removably fixed to the blade support, preferably to the cover thereof, for example via a breakable connector.

The blade protector may be used to prevent inadvertent access to the blade for instance during storage, transport or mounting of the blade unit to the dermatome. As such, the blade may be protected from objects which could detriment its sharpness and/or a user may be protected from being cut by the blade. Additionally or alternatively, the blade protector may reduce the possibility of the dermatome blade damaging a packaging, such as a sterile packaging, thereby reducing the chance of such optionally sterile packaging failing.

As an alternative to a breakable connecting, a snap-fit connection may be used.

In one alternative, the snap fit connection may be arranged so that after removal of the blade protector, it can not be replaced. Accordingly, the snap fit connection, or any other connection type used, may be a single disconnect connection. It is noted that the blade protector may alternatively or additionally be removably fixed to the clamping element, or to the blade support in general.

In another alternative, the snap fit connection may be arranged to allow reconnecting the blade protector, thereby allowing safe disposal of the dermatome blade unit.

In case the blade protector is fixed using a breakable connection, the blade protector may be made integrally with the component to which it is removably fixed. Alternatively, also in the case of using another type of connection, the blade protector may be manufactured as a separated component, which is removably fixed to the blade support, the cover and/or the clamping element after manufacturing.

A breakable connection may be combined with a snap-fit connection, wherein the blade protector may be removed from the blade before use by breaking the breakable connection, and replaced using the snap-fit connection for safe disposal after use.

The invention also relates to a kit of replaceable dermatome blade units as described in the previous paragraphs, wherein the attachment heights of a first dermatome blade units is larger than the attachment height of a second dermatome blade unit.

In such a kit, a thickness of the cover and/or the clamping element can vary from the first to the second dermatome blade unit, in order to provide a corresponding difference in attachment height.

The difference in attachment height results in, as described before, a difference in slice thickness when the blade units are used on a dermatome. By varying the thicknesses of the cover and/or clamping element the attachment height may be varied relatively elegantly. Moreover, the clamping element and/or cover may form a sliding surface for the blade at the desired attachment height. By varying the thickness of the respective component(s), the slide surface may be disposed on the desired height. Moreover, when the thickness of the respective component(s) is used to define the attachment height, it may be clearly visible from the blade unit which attachment height it provides.

In another embodiment of the kit, a total thickness of the cover and the clamping element and the dermatome blade of each dermatome blade unit is defined in the first direction, and wherein the total thickness of the first dermatome blade unit is equal to the total thickness of the second dermatome blade unit.

Accordingly, all blade units have the same total thickness, regardless of their respective blade heights. As such, it may be relatively easy to integrate blade units with different blade heights in a dermatome or dermatome head.

An alternative would be to only vary the thickness of the cover or the clamping element, in particular the one component comprising the connection means, while keeping the clamping element at the same thickness for every dermatome blade unit, while still alternating the difference in attachment height. In this alternative, the single thickness component, e.g. the clamping element or the cover, may benefit from mass production.

The invention also relates to a dermatome comprising a handle and a dermatome head attached to the handle, the dermatome head comprising a guide surface for guiding the dermatome head across skin in use of the dermatome, a dermatome blade unit as described in the previous paragraphs, wherein the dermatome blade unit is attached releasably to the dermatome head using the connecting means of the dermatome blade unit and corresponding connecting means of the dermatome head.

Accordingly, a dermatome is obtained which may cut skin grafts of different thicknesses depending on which dermatome blade unit is used. Such a dermatome has the additional advantage that it is relatively easy to clean, as the dermatome head may be kept relatively elegant. In particular, no moving parts are needed in order to set the cutting height. In fact, the cutting height may be varied by selecting a suitable blade unit.

In a practical embodiment of the dermatome, the connecting means of the dermatome head are stationary with respect to the guide surface of the dermatome head. Accordingly, the thickness of the skin graft can be varied only by the attachment height of the blade unit used. Moreover, when the connecting means are stationary with respect to the guide surface, no added complexity is needed for a blade height adjustment mechanism.

In another embodiment of the dermatome, the dermatome head is attached releasably to the handle.

Releasing the dermatome head, may make cleaning the dermatome relatively easy. Moreover, using releasable dermatome heads allows selecting dermatome heads for suitable purposes, for instance for selecting a grafting thickness or grafting width. Accordingly, the dermatome heads may be designed relatively simply, because it is no longer required that a dermatome head allows for different thicknesses and/or widths. Due to the simple design, cleaning may be even more easy. Additionally or alternatively, the simple design may allow using the dermatome head in its entirety as a single use product at relatively low cost.

It is noted that the same or similar advantages may be achieved using a dermatome as described herein but without the blade unit as described herein. Instead, any suitable blade may be used, for instance one permanently connected to the dermatome head, or a replaceable blade as is known from the prior art.

In yet another embodiment of the dermatome, the handle is provided with a drive mechanism for driving a dermatome blade of a dermatome blade unit attached to the dermatome head in a direction transversal to a grafting direction of the dermatome. The drive mechanism is an example of a drive means as mentioned above.

By providing the drive mechanism in the handle, the dermatome head may be kept relatively elegant and/or simple. As such, the dermatome head may be easier to clean and or less costly to replace. The drive mechanism may be a motor.

If no dermatome blade unit is used, the drive mechanism may be used to drive another type of blade of the dermatome.

In yet another embodiment of the dermatome, the handle of the dermatome comprises at least one battery in an embodiment of the dermatome. Practically, the battery may be used to power the drive mechanism.

Accordingly, the head of the dermatome may be kept relatively simple.

As an alternative, the drive mechanism may be powered by an external power source, such as a pneumatic power source or an electric power source. To this end, the drive mechanism may comprise a suitable power input, such as a pneumatic power input or an electric power input.

The drive mechanism is an example of a drive means introduced above.

The invention also relates to a dermatome kit as described above, and a further dermatome head, the further dermatome head is releasably attachable to the handle in order to replace the dermatome head, wherein a width of the dermatome head as defined in the direction transversal to the grafting direction is larger than a width of the further dermatome head as defined in the direction transversal to the grafting direction.

Using such a kit, a user may select a different dermatome head to achieve a desired width of the skin graft made by moving the dermatome in the grafting direction. By using dermatome heads of different width, skin grafts of the required size may be cut with a relatively simple dermatome head, which may thus be cleaned relatively easily or which may be discarded and replaced at relatively low costs.

The dermatome head and/or the further dermatome may have any of the above described features of the dermatome head, alone or in any suitable combination.

The invention also relates to a dermatome head as described above, for instance in relation to the dermatome or the kit of parts of a dermatome and the further dermatome head, and a dermatome blade unit as described above.

The components of the kit of parts may have any of the above described features, alone or in any suitable combination.

The invention will be further elucidated with reference to the attached drawings, in which:
Figures 1A and 1B respectively show schematically a perspective view and a blow up view of a dermatome;
Figures 2A and 2B schematically show cross sections of two different dermatome blade units;
Figures 3A and 3B respectively show schematically a perspective view and a blow up view of a dermatome blade unit;
Figures 4A and 4B respectively show schematically a perspective view and a blow up view of a dermatome blade unit with a blade protector; and
Figure 5 schematically shows a dermatome blade unit with a blade protector.

Throughout the figures, like elements will be referred to using like reference numerals. Like elements across different embodiments will be referred to using reference numerals increased by 100 (one hundred).

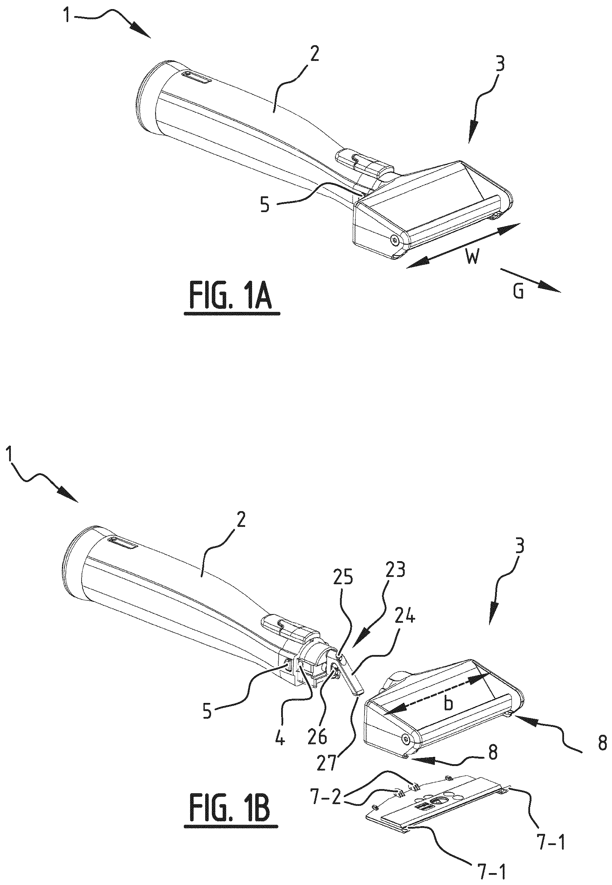

Figures 1A and 1B show a dermatome 1 which comprises a handle 2 and a head 3. The dermatome 1 defines a grafting direction G and a width direction W transversal thereto. The handle 2 is provided with a set of resilient hooks 4 which can be retracted by pressing a button 5 associated with the hooks 4. The hooks extend from the handle 2 of the dermatome 1 for engaging the head 3. The head 3 has corresponding apertures (not visible) in which the hooks 4 engage when the head 3 is connected to the handle 2. Accordingly, the head 3 can be removed from and fixed to the handle 2 by suitable operation of the hooks 4. Accordingly, different heads 3 with similar apertures may be attached to the same handle 2. In particular, heads 3 of different widths b may be used as desired. Figure 1B partially shows drive means 23, which comprise an arm 24 which is pivotable about a joint 25 and in rotation about the joint 25 by an eccentrically mounted cam 26. The arm 24 comprises at its end a pin 27 of a relatively hard material, such as a hardened metal, for engagement of the dermatome blade 9 described below. Figure 1B also shows a dermatome blade unit 6 which in its entirety can be connected to and released from the dermatome head 3. For this purpose, the dermatome blade unit 6 is provided with connection means 7-1, 7-2 (collectively referred to as 7). The head 3 is provided with corresponding connecting means 8. Further details of the dermatome blade unit 6 will be described below with reference to the following figures.

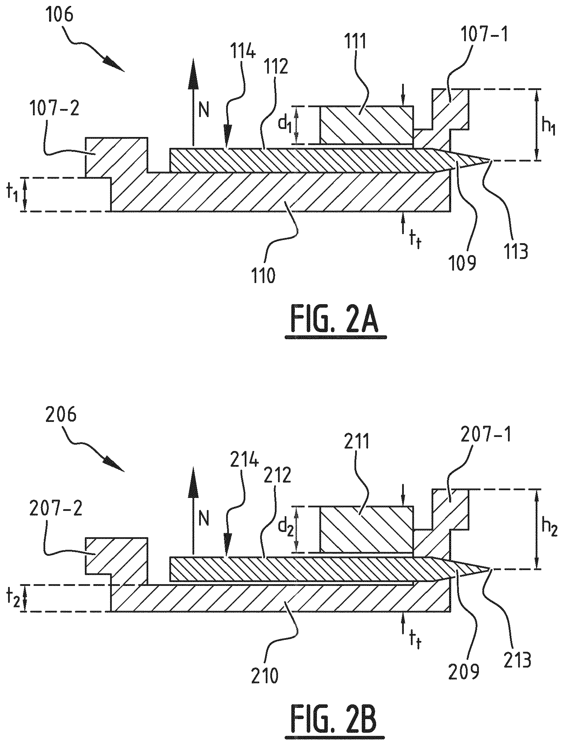

Figures 2A and 2B each show a dermatome blade unit 106, 206 which could be used in the dermatome 1 of figures 1A - 1B. the dermatome blade unit 106, 206 has a dermatome blade 109, 209 and a blade support, which herein consists of a cover 110, 210 and a clamping element 111, 211, although other components could also be part of the blade support. The dermatome blade 109, 209 has a main body 112, 212 and a cutting edge 113, 213. The main body 112, 212 defines a main plane 114, 214 and a direction N normal thereto. The cover 110, 210 comprises connecting means 107-1, 207-1 and 107-2, 207-2, which are schematically represented in figures 2A and 2B. Taking one of the connecting means 107-1, 207-1 as a reference, an attachment height h₁, h₂ for the dermatome blade 109, 209 can be defined. The attachment height h₁, h₂ defines the position of the dermatome blade 109, 209 with respect to the head 3 once the dermatome blade unit 106, 206 has been connected to the head 3. Comparing the dermatome blade unit 106 of figure 2A to that 206 of figure 2B, the attachment heights h₁, h₂ are different. The difference is caused by varying a thickness t₁, t₂ of the cover 110, 210, so that the dermatome blade 109, 209 is held at the desired attachment height h₁, h₂ with respect to the connecting means 107-1, 207-1. The clamping element 111, 211 has a thickness d₁, d₂ which is varied in correspondence with the thickness t₁, t₂ of the cover 110, 210, so that a total thickness tₜ of the dermatome blade unit 106, 206 remains constant even when the attachment height h₁, h₂ is varied. The thicknesses t₁, t₂, tₜ, d₁, d₂ and the attachment height h₁, h₂ are defined in the normal direction N.

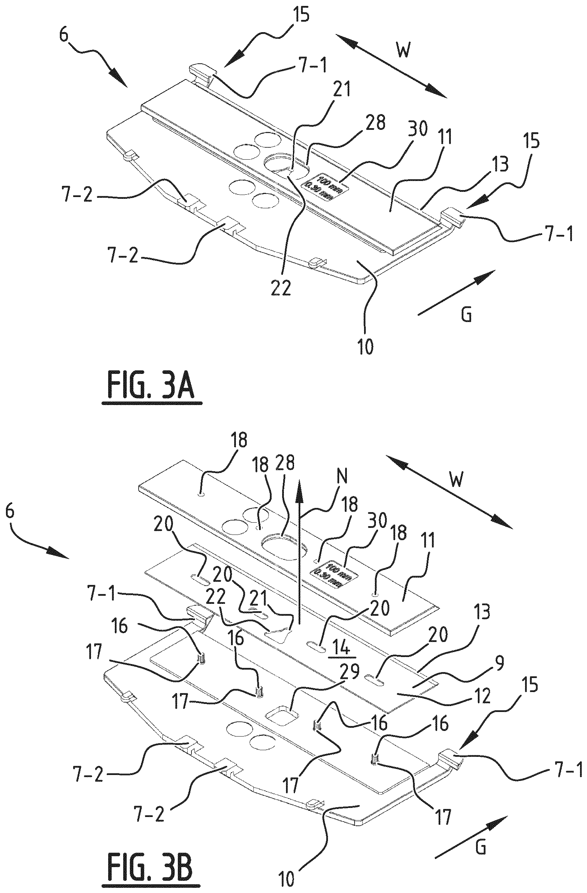

Figures 3A and 3B show the dermatome blade unit 6 of figures 1A and 1B in more detail. In particular, it can be seen that both types connecting means 7-1, 7-2 comprise hooks 7-1, 7-2 which extend towards opposites sides from the cover 10. Accordingly, the cover 10 can be suspended between cooperating connecting means 8 in the head 3 from the connecting means 7. The connecting means 7 are made from a slightly resilient material, in this example plastic, so that they can deform and snap into place for locking the cover 10 in place in the head 3. The front of the connecting means 7-1 functions at the same time as a guide surface 15 for guiding the dermatome blade unit 6 across the skin in use of the dermatome 1. The cover 10 is provided with a series of pillars 16 which extend in the normal direction N. Each pillar 16 is provided with a stop 17, in this example embodied as a wider bottom part of the pillar 16 near the main body of the cover 10. The clamping element 11 is provided with corresponding apertures 18, in which the pillars 16 can be pressed with some force. The pillars 16 and apertures 18 are shaped such that a clamping action keeps the clamping element 11 in place with respect to the cover 10. The dermatome blade 9 is arranged between the cover 10 and the clamping element 11. The dermatome blade 9 comprises slots 19 which correspond to the pillars 16 of the cover 10. The slots 19 are somewhat wider than the diameter of the pillars 16, so that the dermatome blade 9 can slide in the width direction W width respect to the cover 10 and the clamping element 11. In motion of the blade 9 with respect to the cover 10, the slots 20 move in their longitudinal direction while the pillars 16 remain stationary and in the slots 20. As such, the pillars 16 thus move along the length of their respective slots 20, as seen from the reference point of the pillars 16. The blade 9 further includes an aperture 21, 22, which has an engagement part 21 which is relatively narrow, and a wider receiving part 22. The inside surface of the engagement part 21 forms an engagement surface for engagement by a drive means 23 of the dermatome 1. The receiving part 22 allows, due to its larger dimensions, easy insertion of the drive means 23, in particular the pin 27 thereof, into the aperture 21, 22. The pin 27 can then, during placing of dermatome blade unit 6 in the head 3, be moved to the engagement part 21, where it engages the surface of the engagement part 21 for moving the dermatome blade 9 in the width direction reciprocally with respect to the blade support and with respect to the head 3 and handle 2. The cover 10 and the clamping element 11 comprise oversized apertures 28, 29 which align with the apertures 21, 22 of the dermatome blade 9. The oversized apertures allow the pin 27 of the drive means 23 to engage only the dermatome blade 9. The dermatome blade unit 6 is further provided with a marking 30 which indicates the dimensions of the dermatome blade unit 6. In particular, an indication representative of the attachment height h₁, h₂, or the thickness of a skin graft cut with such an attachment height h₁, h₂, may be displayed, so as to allow easily distinguishing different dermatome blade units 6.

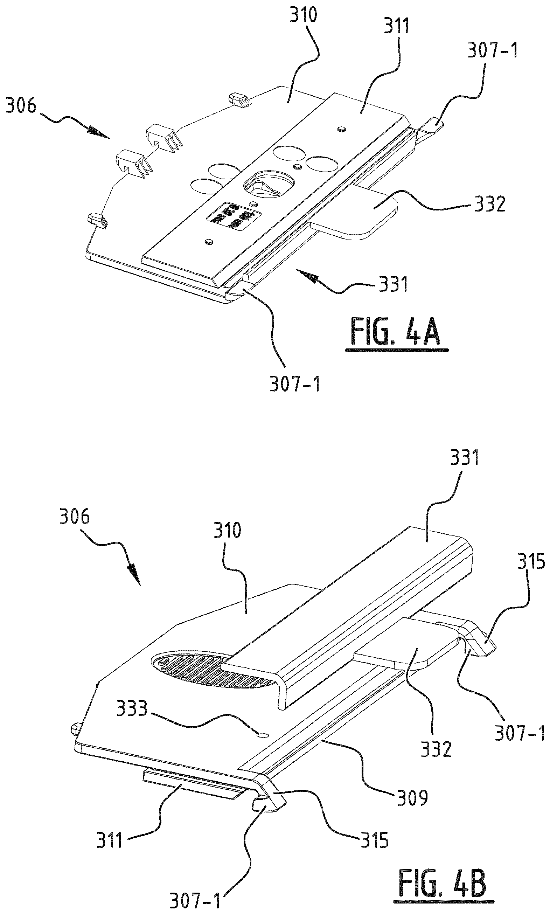

Figures 4A and 4B show different perspective views of a dermatome blade unit 306 which is the same as the above described dermatome blade unit 6, but differs in that it is provided with a blade protector 331, which is removably fixed to the blade support. The blade protector 331 covers the dermatome blade 309 to protect it from inadvertent contact. The blade protector 331 comprises a tab 332 which can be pulled in order to remove the blade protector 331 from the dermatome blade unit 306. The blade protector 331 is fixed by engaging with protrusions (not shown) in apertures 333 in the cover 310.

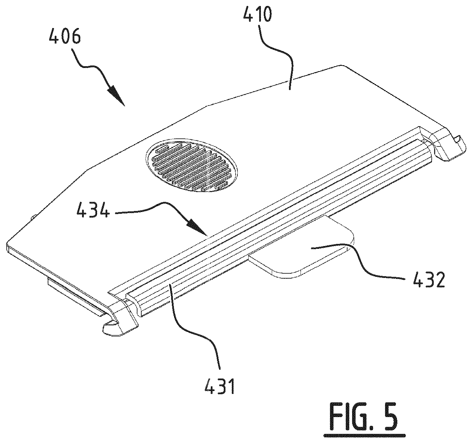

Figure 5 shows a similar dermatome blade unit 406, which differs only in that the blade protector 431 is removably fixed to the cover 410 using a breakable connection along the length of the blade protector 431. It is thus connected along a line 434 to the cover 410. The connection line 434 is made from a relatively thin part of material, in this case plastic, so that it may be broken to remove the blade protector 431, for instance tilting the blade protector 431 with respect to the cover 310, e.g. by pulling at the tab 432.

Although the invention has been described above with reference to specific embodiments and examples, the invention is not limited thereto. Instead, the invention is also defined by the following claims.

## Claims

1. Dermatome blade unit (6, 106, 206), comprising:
- a blade support, comprising connecting means (7, 107, 207) for releasably attaching the dermatome blade unit (6, 106, 206) to a handle (2) and/or a dermatome head (3); and
- a dermatome blade (9, 109, 209) attached to the blade support,
wherein a distance in a first direction normal (N) to a main surface (114, 214) of the dermatome blade (9, 109, 209) between the dermatome blade (9, 109, 209) and the connecting means (7, 107, 207) is defined as an attachment height (h₁, h₂),
**characterized in that**
the dermatome blade (9, 109, 209) is moveable with respect to the respective blade support in a direction substantially parallel to a direction in which a cutting edge (113, 213) of the dermatome blade (9, 109, 209) extends.

2. Dermatome blade unit (6, 106, 206) according to the previous claim, wherein the blade support comprises a cover (10, 110, 210) and a clamping element (11, 111, 211), the blade being interposed between the cover (10, 110, 210) and the clamping element (11, 111, 211).

3. Dermatome blade unit (6, 106, 206) according to the previous claim, wherein one of the cover (10, 110, 210) and the clamping element (11, 111, 211) comprises male connecting units and the other of the cover (10, 110, 210) and the clamping element (11, 111, 211) comprises corresponding female connecting units, the male and female connecting units being configured to engage each other in order to lock the clamping element (11, 111, 211) in place with respect to the cover (10, 110, 210).

4. Dermatome blade unit (6, 106, 206) according to any of the preceding claims, wherein each dermatome blade comprises an engagement surface, configured for engagement by drive means (23) of a dermatome (1) for moving the dermatome blade (9, 109, 209) with respect to the blade support.

5. Dermatome blade unit (6, 106, 206) according to the previous claim, wherein the engagement surface is formed by an aperture (21, 22) and/or recess in the dermatome blade.

6. Dermatome blade unit (6, 106, 206) according to any of the preceding claims, wherein each blade support comprises a guide surface (15) for guiding the dermatome blade unit (6, 106, 206) across skin during use of the respective dermatome blade unit (6, 106, 206) in a dermatome (1).

7. Dermatome blade unit (6, 106, 206) according to any of the preceding claims, wherein each dermatome blade unit (6, 106, 206) comprises a removable blade protector (331, 431), removably fixed to the blade support, preferably to the cover (10, 110, 210) thereof, for example via a breakable connector.

8. Kit of replaceable dermatome blade units (6, 106, 206), comprising at least a first dermatome blade unit (6, 106, 206) according to claim 1 and a second dermatome blade unit (6, 106, 206) according to claim 1, wherein the attachment height (hi) of the first dermatome blade unit (106) is larger than the attachment height (h₂) of the second dermatome blade unit (206).

9. Kit of replaceable dermatome blade units (6, 106, 206), comprising at least a first dermatome blade unit (6, 106, 206) according to any of claims 2-7 and a second dermatome blade unit (6, 106, 206) according to any of claims 2-7, wherein the attachment height (h 1) of the first dermatome blade unit (106) is larger than the attachment height (h2) of the second dermatome blade unit, wherein a thickness (t₁, t₂, d₁, d₂) of the cover (10, 110, 210) and/or the clamping element (11, 111, 211) varies from the first to the second dermatome blade unit (6, 106, 206), in order to provide a corresponding difference in attachment height (h₁, h₂).

10. Kit of replaceable dermatome blade units (6, 106, 206) according to the previous claim, wherein a total thickness (tₜ) of the cover (10, 110, 210) and the clamping element (11, 111, 211) and the dermatome blade of each dermatome blade unit (6, 106, 206) is defined in the first direction, and wherein the total thickness (tt) of the first dermatome blade unit (6, 106, 206) is equal to the total thickness (tt) of the second dermatome blade unit (6, 106, 206).

11. Dermatome (1) comprising:
- a handle (2);
- a dermatome head (3) attached to the handle (2), the dermatome head (3) comprising a guide surface (15) for guiding the dermatome head (3) across skin in use of the dermatome (1); and
- a dermatome blade unit (6, 106, 206) according to any of claims 1 - 7,
wherein the dermatome blade unit (6, 106, 206) is attached releasably to the dermatome head (3) using the connecting means of the dermatome blade unit (6, 106, 206) and corresponding connecting means of the dermatome head (3).

12. Dermatome (1) according to the previous claim, wherein the connecting means of the dermatome head (3) are stationary with respect to the guide surface (15) of the dermatome head (3), and/or wherein the dermatome head (3) is attached releasably to the handle (2).

13. Dermatome (1) according to any of claims 11 - 12, wherein the handle (2) is provided with a drive mechanism (23) for driving a dermatome blade of a dermatome blade unit (6, 106, 206) attached to the dermatome head (3) in a direction transversal to a grafting direction of the dermatome (1).

14. Dermatome (1) according to any of claims 11 - 13, wherein the handle (2) comprises at least one battery, wherein optionally the drive mechanism (23) is powered by the at least one battery.

15. Dermatome kit comprising:
a dermatome (1) according to any of claims 11 - 14, and a further dermatome head (3), the further dermatome head (3) being releasably attachable to the handle (2) in order to replace the dermatome head (3), wherein a width (b) of the dermatome head (3) as defined in the direction substantially transversal to the grafting direction is larger than a width (b) of the further dermatome head (3) as defined in the direction transversal to the grafting direction.

## Patentansprüche

1. Dermatomklingeneinheit (6, 106, 206), umfassend:
- einen Klingenträger, umfassend Verbindungsmittel (7, 107, 207)zum lösbaren Befestigen der Dermatomklingeneinheit (6, 106, 206) an einem Griff (2) und/oder einem Dermatomkopf (3); und
- eine Dermatomklinge (9, 109, 209), die an dem Klingenträger befestigt ist,
wobei ein Abstand in einer ersten Richtung normal (N) zu einer Hauptoberfläche (114, 214) der Dermatomklinge (9, 109, 209) zwischen der Dermatomklinge (9, 109, 209) und dem Verbindungsmittel (7, 107, 207) als eine Befestigungshöhe (h₁, h₂) definiert ist,
**dadurch gekennzeichnet, dass**
die Dermatomklinge (9, 109, 209) hinsichtlich dem jeweiligen Klingenträger in einer Richtung im Wesentlichen parallel zu einer Richtung bewegbar ist, in der sich eine Schneidkante (113, 213) der Dermatomklinge (9, 109, 209) erstreckt.

2. Dermatomklingeneinheit (6, 106, 206) nach dem vorstehenden Anspruch, wobei der Klingenträger eine Abdeckung (10, 110, 210) und ein Klemmelement (11, 111, 211) umfasst, wobei die Klinge zwischen der Abdeckung (10, 110, 210) und dem Klemmelement (11, 111, 211) eingefügt ist.

3. Dermatomklingeneinheit (6, 106, 206) nach dem vorstehenden Anspruch, wobei eines von der Abdeckung (10, 110, 210) und dem Klemmelement (11, 111, 211) männliche Verbindungseinheiten umfasst und das andere von der Abdeckung (10, 110, 210) und dem Klemmelement (11, 111, 211) entsprechende weibliche Verbindungseinheiten umfasst, wobei die männlichen und weiblichen Verbindungseinheiten konfiguriert sind, um einander in Eingriff zu nehmen, um das Klemmelement (11, 111, 211) hinsichtlich der Abdeckung (10, 110, 210) an seinem Platz zu arretieren.

4. Dermatomklingeneinheit (6, 106, 206) nach einem der vorstehenden Ansprüche, wobei jede Dermatomklinge eine Eingriffsoberfläche umfasst, die für den Eingriff durch Antriebsmittel (23) eines Dermatoms (1) zum Bewegen der Dermatomklinge (9, 109, 209) hinsichtlich dem Klingenträger konfiguriert ist.

5. Dermatomklingeneinheit (6, 106, 206) nach dem vorstehenden Anspruch, wobei die Eingriffsoberfläche durch eine Öffnung (21, 22) und/oder Aussparung in der Dermatomklinge ausgebildet ist.

6. Dermatomklingeneinheit (6, 106, 206) nach einem der vorstehenden Ansprüche, wobei jeder Klingenträger eine Führungsoberfläche (15) zum Führen der Dermatomklingeneinheit (6, 106, 206) über eine Haut hinweg während einer Verwendung der jeweiligen Dermatomklingeneinheit (6, 106, 206) in einem Dermatom (1) umfasst.

7. Dermatomklingeneinheit (6, 106, 206) nach einem der vorstehenden Ansprüche, wobei jede Dermatomklingeneinheit (6, 106, 206) einen abnehmbaren Klingenschutz (331, 431) umfasst, der an der Klingenhalterung, vorzugsweise an der Abdeckung (10, 110, 210) davon, abnehmbar fixiert ist, beispielsweise über ein abbrechbares Verbindungsstück.

8. Kit aus austauschbaren Dermatomklingeneinheiten (6, 106, 206), umfassend mindestens eine erste Dermatomklingeneinheit (6, 106, 206) nach Anspruch 1 und eine zweite Dermatomklingeneinheit (6, 106, 206) nach Anspruch 1, wobei die Befestigungshöhe (h₁) der ersten Dermatomklingeneinheit (106) größer als die Befestigungshöhe (h₂) der zweiten Dermatomklingeneinheit (206) ist.

9. Kit aus austauschbaren Dermatomklingeneinheiten (6, 106, 206), umfassend mindestens eine erste Dermatomklingeneinheit (6, 106, 206) nach einem der Ansprüche 2 bis 7 und eine zweite Dermatomklingeneinheit (6, 106, 206) nach einem der Ansprüche 2 bis 7, wobei die Befestigungshöhe (h 1) der ersten Dermatomklingeneinheit (106) größer als die Befestigungshöhe (h2) der zweiten Dermatomklingeneinheit ist, wobei eine Dicke (t₁, t₂, d₁, d₂) der Abdeckung (10, 110, 210) und/oder des Klemmelements (11, 111, 211) von der ersten zu der zweiten Dermatomklingeneinheit (6, 106, 206) variiert, um einen entsprechenden Unterschied in der Befestigungshöhe (h₁, h₂) bereitzustellen.

10. Kit aus austauschbaren Dermatomklingeneinheiten (6, 106, 206) nach dem vorstehenden Anspruch, wobei eine Gesamtdicke (tₜ) der Abdeckung (10, 110, 210) und des Klemmelements (11, 111, 211) und der Dermatomklinge jeder Dermatomklingeneinheit (6, 106, 206) in der ersten Richtung definiert ist, und wobei die Gesamtdicke (tt) der ersten Dermatomklingeneinheit (6, 106, 206) gleich der Gesamtdicke (tt) der zweiten Dermatomklingeneinheit (6, 106, 206) ist.

11. Dermatom (1), umfassend:
- einen Griff (2);
- einen Dermatomkopf (3), der an dem Griff (2) befestigt ist, der Dermatomkopf (3) umfassend eine Führungsoberfläche (15) zum Führen des Dermatomkopfs (3) über die Haut hinweg bei der Verwendung des Dermatoms (1); und
- eine Dermatomklingeneinheit (6, 106, 206) nach einem der Ansprüche 1 bis 7,
wobei die Dermatomklingeneinheit (6, 106, 206) unter Verwendung der Verbindungsmittel der Dermatomklingeneinheit (6, 106, 206) und entsprechender Verbindungsmittel des Dermatomkopfs (3) an dem Dermatomkopf (3) lösbar befestigt ist.

12. Dermatom (1) nach dem vorstehenden Anspruch, wobei die Verbindungsmittel des Dermatomkopfs (3) hinsichtlich der Führungsoberfläche (15) des Dermatomkopfs (3) stationär sind und/oder wobei der Dermatomkopf (3) an dem Griff (2) lösbar befestigt ist.

13. Dermatom (1) nach einem der Ansprüche 11 bis 12, wobei der Griff (2) mit einem Antriebsmechanismus (23) zum Antreiben einer Dermatomklinge einer Dermatomklingeneinheit (6, 106, 206), die an dem Dermatomkopf (3) befestigt ist, in einer Richtung quer zu einer Transplantationsrichtung des Dermatoms (1) versehen ist.

14. Dermatom (1) nach einem der Ansprüche 11 bis 13, wobei der Griff (2) mindestens eine Batterie umfasst, wobei optional der Antriebsmechanismus (23) durch die mindestens eine Batterie betrieben wird.

15. Dermatomkit, umfassend:
ein Dermatom (1) nach einem der Ansprüche 11 bis 14 und einen weiteren Dermatomkopf (3), wobei der weitere Dermatomkopf (3) an dem Griff (2) lösbar befestigt werden kann, um den Dermatomkopf (3) zu ersetzen, wobei eine Breite (b) des Dermatomkopfs (3) in der Richtung im Wesentlichen quer zu der Transplantationsrichtung größer als eine Breite (b) des weiteren Dermatomkopfs (3) in der Richtung quer zu der Transplantationsrichtung ist.

## Revendications

1. Unité de lame de dermatome (6, 106, 206), comprenant :
- un support de lame, comprenant des moyens de liaison (7, 107, 207) permettant de fixer de manière libérable l'unité de lame de dermatome (6, 106, 206) à une poignée (2) et/ou une tête de dermatome (3) ; et
- une lame de dermatome (9, 109, 209) fixée au support de lame,
dans laquelle une distance dans une première direction normale (N) à une surface principale (114, 214) de la lame de dermatome (9, 109, 209) entre la lame de dermatome (9, 109, 209) et le moyen de liaison (7, 107, 207) est définie comme une hauteur de fixation (h₁, h₂),
**caractérisée en ce que**
la lame de dermatome (9, 109, 209) est mobile par rapport au support de lame respectif dans une direction sensiblement parallèle à une direction dans laquelle un bord de coupe (113, 213) de la lame du dermatome (9, 109, 209) s'étend.

2. Unité de lame de dermatome (6, 106, 206) selon la revendication précédente, dans laquelle le support de lame comprend un
couvercle (10, 110, 210) et un élément de serrage (11, 111, 211), la lame étant interposée entre le couvercle (10, 110, 210) et l'élément de serrage (11, 111, 211).

3. Unité de lame de dermatome (6, 106, 206) selon la revendication précédente, dans laquelle l'un parmi le couvercle (10, 110, 210) et l'élément de serrage (11, 111, 211) comprend des unités de liaison mâles et l'autre parmi le couvercle (10, 110, 210) et l'élément de serrage (11, 111, 211) comprend des unités de liaison femelles correspondantes, les unités de liaison mâles et femelles étant conçues pour venir en prise l'une avec l'autre afin de verrouiller l'élément de serrage (11, 111, 211) en place par rapport au couvercle (10, 110, 210).

4. Unité de lame de dermatome (6, 106, 206) selon l'une quelconque des revendications précédentes, dans laquelle chaque lame de dermatome comprend une surface de mise en prise, conçue pour venir en prise avec les moyens d'entraînement (23) d'un dermatome (1) pour entraîner en mouvement la lame de dermatome (9, 109, 209) par rapport au support de lame.

5. Unité de lame de dermatome (6, 106, 206) selon la revendication précédente, dans laquelle la surface de mise en prise est formée par une ouverture (21, 22) et/ou un renfoncement dans la lame de dermatome.

6. Unité de lame de dermatome (6, 106, 206) selon l'une quelconque des revendications précédentes, dans laquelle chaque support de lame comprend une surface de guide (15) permettant de guider l'unité de lame de
dermatome (6, 106, 206) sur la peau pendant l'utilisation de l'unité de lame de dermatome (6, 106, 206) respective dans un dermatome (1).

7. Unité de lame de dermatome (6, 106, 206) selon l'une quelconque des revendications précédentes, dans laquelle chaque unité de lame de dermatome (6, 106, 206) comprend un protecteur de lame amovible (331, 431), fixé de manière amovible au support de lame, de préférence au couvercle (10, 110, 210) de celui-ci, par exemple par l'intermédiaire d'un élément de liaison cassable.

8. Kit d'unités de lames de dermatome remplaçables (6, 106, 206), comprenant au moins une première unité de lame de dermatome (6, 106, 206) selon la revendication 1 et une seconde unité de lame de
dermatome (6, 106, 206) selon la revendication 1, dans lequel la hauteur de fixation (h₁) de la première unité de lame de dermatome (106) est plus grande que la hauteur de fixation (h₂) de la seconde unité de lame de dermatome (206).

9. Kit d'unités de lames de dermatome remplaçables (6, 106, 206), comprenant au moins une première unité de lame de dermatome (6, 106, 206) selon l'une quelconque des revendications 2 à 7 et une seconde unité de lame de dermatome (6, 106, 206) selon l'une quelconque des revendications 2 à 7, dans lequel la hauteur de fixation (h 1) de la première unité de lame de dermatome (106) est plus grande que la hauteur de fixation (h2) de la seconde unité de lame de dermatome, dans lequel une épaisseur (t₁, t₂, d₁, d₂) du couvercle (10, 110, 210) et/ou de l'élément de serrage (11, 111, 211) varie entre la première et la seconde unité de lame de dermatome (6, 106, 206), afin de fournir une différence correspondante dans la hauteur de fixation (h₁, h₂).

10. Kit d'unités de lames de dermatome remplaçables (6, 106, 206) selon la revendication précédente, dans lequel une épaisseur totale (tt) du couvercle (10, 110, 210) et de l'élément de serrage (11, 111, 211) et de la lame de dermatome de chaque unité de lame de dermatome (6, 106, 206) est définie dans la première direction, et dans lequel l'épaisseur totale (tt) de la première unité de lame de dermatome (6, 106, 206) est égale à l'épaisseur totale (tₜ) de la seconde unité de lame de dermatome (6, 106, 206).

11. Dermatome (1), comprenant:
- une poignée (2) ;
- une tête de dermatome (3) fixée à la poignée (2), la tête de dermatome (3) comprenant une surface de guide (15) permettant de guider la tête de dermatome (3) sur la peau pendant l'utilisation du dermatome (1) ; et
- une unité de lame de dermatome (6, 106, 206) selon l'une quelconque des revendications 1 à 7,
dans lequel l'unité de lame de dermatome (6, 106, 206) est fixée de manière libérable à la tête de dermatome (3) à l'aide des moyens de liaison de l'unité de lame de dermatome (6, 106, 206) et des moyens de liaison correspondants de la tête de dermatome (3).

12. Dermatome (1) selon la revendication précédente, dans lequel les moyens de liaison de la tête de dermatome (3) sont fixes par rapport à la surface de guide (15) de la tête de dermatome (3), et/ou dans lequel la tête de dermatome (3) est fixée de manière libérable à la poignée (2).

13. Dermatome (1) selon l'une quelconque des revendications 11 à 12, dans lequel la poignée (2) est dotée d'un mécanisme d'entraînement (23) permettant d'entraîner une lame de dermatome d'une unité de lame de dermatome (6, 106, 206) fixée à la tête de dermatome (3) dans une direction transversale à la direction de greffage du dermatome (1).

14. Dermatome (1) selon l'une quelconque des revendications 11 à 13, dans lequel la poignée (2) comprend au moins une batterie, dans lequel éventuellement le mécanisme d'entraînement (23) est alimenté par l'au moins une batterie.

15. Kit de dermatome comprenant:
un dermatome (1) selon l'une quelconque des revendications 11 à 14, et une autre tête de dermatome (3), l'autre tête de dermatome (3) pouvant être fixée de manière libérable à la poignée (2) afin de remplacer la tête de dermatome (3), dans lequel une largeur (b) de la tête de dermatome (3) telle que définie dans la direction sensiblement transversale à la direction de greffage est plus grande qu'une largeur (b) de l'autre tête de dermatome (3) telle que définie dans la direction transversale à la direction de greffage.
